# EUROPEAN PATENT SPECIFICATION

(11) **EP 2 039 297 B1**
(45) Date of publication and mention of the grant of the patent: **23.11.2016**
(21) Application number: 08016453.6
(22) Date of filing: 18.09.2008
(51) Int. Cl.: A61B 8/12, A61B 8/08, G01S 15/89, G01N 29/26

(54) **Ultrasound diagnostic apparatus**
Diagnostisches Ultraschallgerät
Appareil de diagnostic à ultrasons

(30) Priority: 21.09.2007 JP 2007245785
(43) Date of publication of application: 25.03.2009
(73) Proprietor: Olympus Corporation, Tokyo 192-8507 (JP)
(72) Inventor: Hibi, Yasushi, Hachioji-shi, Tokyo 192-8507 (JP)
(74) Representative: von Hellfeld, Axel

(56) References cited:
- EP-A- 0 139 574
- WO-A-2004/064614
- GB-A- 2 246 632
- US-A- 5 186 176
- US-A- 5 186 177
- US-A1- 2005 085 730

## Description

### BACKGROUND OF THE INVENTION

### 1. Field of the Invention

The present invention relates to an ultrasound diagnostic apparatus, and particularly relates to an ultrasound diagnostic apparatus including an ultrasound endoscope having a plurality of transducer groups at a distal end portion of a probe.

### 2. Description of the Related Art

In recent years, a method has become widespread, in which an ultrasound endoscope is inserted into a living body, a lesion in the body is found from its optical image, the lesion is irradiated with ultrasound, and an ultrasound tomogram of the lesion is diagnosed from the reflected wave. Further, a method is carried out, in which a cell is aspirated by being punctured while being visually recognized under guide of an optical image/ultrasound tomogram with use of a puncture needle, and confirmed diagnosis is performed by the aspirated cell.

When the diagnosis is carried out, a tissue needs to be accurately extracted. As an extraction method, there are a method in which a needle is inserted into a tumor under B mode guide of the ultrasound image and the tutor is reliably extracted, and the like. In this case, in order to diagnose a lesion in a lumen such as an inside of a body, radial scanning capable of scanning a whole of the inside of the lumen is suitable at first. An operator diagnoses the lesion by using a radial scanning intracavital ultrasound endoscope. In order to carry out confirmed diagnosis after the position of the lesion is confirmed, the lesion needs to be searched for again with a convex scanning intracavital ultrasound endoscope, and the convex scanning intracavital ultrasound endoscope is inserted into a patient again. Accordingly, the patient needs to swallow the intracavital ultrasound endoscope twice, and the patient is compelled to suffer pain.

Therefore, each of Japanese Patent Application Laid-Open Publication No. 8-56948 and Japanese Patent Application Laid-Open Publication No. 2004-135693 discloses a biplane ultrasound endoscope in which a transducer group for radial scanning and a transducer group for convex scanning are disposed close to each other at a probe so that scanning directions intersect each other. According to the ultrasound endoscope, sectional images in different directions can be observed with one scope.

Further, Japanese Patent Application Laid-Open Publication No. 2002-177278 discloses an ultrasound diagnostic apparatus in which a probe having three transducers is used, and a transducer switching circuit for switching a transducer for use is included so that the respective transducers share a transmission and reception section.

The biplane ultrasound diagnostic apparatus using two transducer groups which is disclosed in each of Japanese Patent Application Laid-Open Publication No. 8-56948 and Japanese Patent Application Laid-Open Publication No. 2004-135693 requires respective sound ray synthesis tables, transmission and reception sections, image generating sections and the like corresponding to the respective transducer groups, and the apparatus configuration is complicated.

Further, the ultrasound diagnostic apparatus disclosed in Japanese Patent Application Laid-Open Publication No. 2002-0177278 only discloses that the probe having a plurality of transducer groups in the same shapes and of the same drive methods is used, and the same control device or the like is used by being switched.

US 5,186,176 relates to an ultrasonic diagnosis apparatus comprising an ultrasonic probe like a biplane probe having two ultrasonic transducers, a switch for selecting one of the ultrasonic transducers, a transmitting-receiving system for driving an ultrasonic transducer selected by the switch to transmit and receive ultrasonic waves, a B-mode image producing means responsive to a receive signal from the ultrasonic transducer driven by the transmitting-receiving system for producing an ultrasonic cross-sectional image, a scanning-plane mark generator for generating a scanning-plane mark indicating the positional relationship between the ultrasonic scanning plane formed by the ultrasonic transducer selected by the switch and the other ultrasonic scanning plane formed by the other ultrasonic transducer and a TV monitor for displaying a superimposition image in which the scanning-plane mark generated by the scanning-plane mark generator is superimposed on the ultrasonic cross-sectional image produced by the B-mode image producing system.

EP 0 139 574 A2 refers to an endocavitary ultrasound probe for delivering two ultrasonic views of two different scanning planes of a single region of the body surrounding a cavity in which that probe is inserted, including a substantially longitudinal and cylindrical support, a plurality of elementary transducers forming a first electronic scanning transducer of a first plane of observation, a second transducer, electrical, electronical and mechanical connecting means extending inside that support from said transducers to, on one way, a source of excitation signals and on the other way, a visualisation unit of the back signals, wherein the plurality of transducers of said first scanning transducer is located substantially along a generative line of said support, for scanning a plane surface including substantially the longitudinal axis of said support and wherein said second transducer generates an ultrasonic beam included in a scanning surface which intersects said plane surface.

WO 2004/064614 A2 relates to an ultrasonic probe comprising an elongate structure having a longitudinal axis, a first array of ultrasonic transducer elements extending along an outer surface of the elongate structure in a direction generally parallel to the longitudinal axis, a second array of ultrasonic transducer elements extending along the outer surface of the elongate structure in a direction generally parallel to the longitudinal axis, and a third array of ultrasonic transducer elements extending about the elongate structure in a direction so that it images a plane perpendicular to that imaged by at least one of the first array and the second array, the third array being disposed in a space between the first array and the second array.

The present invention has an object to provide an ultrasound diagnostic apparatus having a plurality of transducer groups with a simple apparatus configuration.

### SUMMARY OF THE INVENTION

In order to attain the above-described object, an ultrasound diagnostic apparatus of the present invention has a plurality of transducer groups which are provided at a distal end portion of a probe, and are configured by a plurality of transducers arranged in a circumferential form or an arc form having a same radius of curvature, and a common sound ray synthesis table which stores control data of the respective transducer groups.

### BRIEF DESCRIPTION OF THE DRAWINGS

Fig. 1 is a configuration diagram of an ultrasound diagnostic apparatus according to an embodiment of the present invention;
Fig. 2 is a perspective view of a probe distal end portion according to the embodiment of the present invention;
Fig. 3A is a front view of the probe distal end portion according to the embodiment of the present invention;
Fig. 3B is a side view of the probe distal end portion according to the embodiment of the present invention;
Fig. 4A is a sectional view showing sound rays of an ELR scanning transducer group according to the embodiment of the present invention;
Fig. 4B is a sectional view showing sound rays of a CLA scanning transducer group according to the embodiment of the present invention;
Fig. 5A is a sectional view showing sound ray synthesis of the ELR scanning transducer group according to the embodiment of the present invention;
Fig. 5B is a sectional view showing sound ray synthesis of the CLA scanning transducer group according to the embodiment of the present invention;
Fig. 6 is a display example of ultrasound images displayed on a display device according to the embodiment of the present invention;
Fig. 7 is a flowchart of scanning of each transducer configuring the transducer groups according to the embodiment of the present invention;
Fig. 8 is a flowchart of scanning of each transducer configuring the transducer groups according to the embodiment of the present invention;
Fig. 9A is a view showing a scanning surface in an ultrasound diagnostic apparatus having two transducer groups scanning within surfaces orthogonal to each other according to the embodiment of the present invention;
Fig. 9B is a view showing a scanning surface in the ultrasound diagnostic apparatus having the two transducer groups scanning within the surfaces orthogonal to each other according to the embodiment of the present invention;
Fig. 9C is a view showing a scanning surface in the ultrasound diagnostic apparatus having the two transducer groups scanning within the surfaces orthogonal to each other according to the embodiment of the present invention;
Fig. 10A is a view showing a scanning surface in an ultrasound diagnostic apparatus having three transducer groups scanning within surfaces orthogonal to one another according to the embodiment of the present invention;
Fig. 10B is a view showing a scanning surface in the ultrasound diagnostic apparatus having the three transducer groups scanning within the surfaces orthogonal to one another according to the embodiment of the present invention;
Fig. 10C is a view showing a scanning surface in the ultrasound diagnostic apparatus having the three transducer groups scanning within the surfaces orthogonal to one another according to the embodiment of the present invention;
Fig. 11A is a view showing a scanning surface in an ultrasound diagnostic apparatus having five transducer groups scanning within the surfaces orthogonal to one another according to the embodiment of the present invention;
Fig. 11B is a view showing a scanning surface in the ultrasound diagnostic apparatus having the five transducer groups scanning within the surfaces orthogonal to one another according to the embodiment of the present invention;
Fig. 11C is a view showing a scanning surface in the ultrasound diagnostic apparatus having the five transducer groups scanning within the surfaces orthogonal to one another according to the embodiment of the present invention; and
Fig. 12 is a flowchart for explaining the flow of processing of the ultrasound diagnostic apparatus according to the embodiment of the present invention.

### DETAILED DESCRIPTION OF THE PREFERRED EMBODIMENTS

Hereinafter, embodiments of the present invention will be described with reference to the drawings.

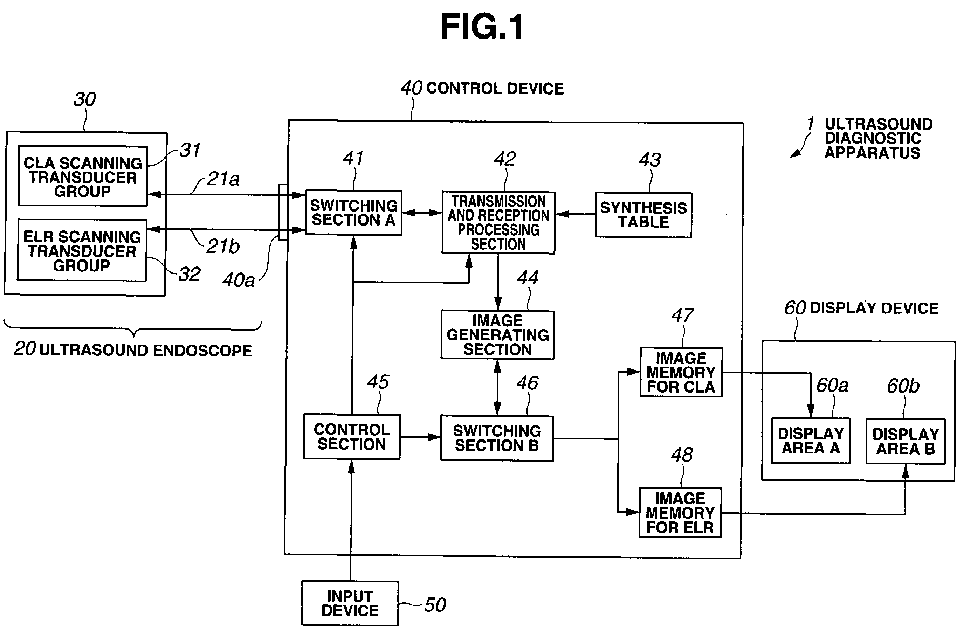

Fig. 1 is a configuration diagram of an ultrasound diagnostic apparatus 1 according to an embodiment of the present invention. The ultrasound diagnostic apparatus 1 of the present embodiment includes an ultrasound endoscope 20, a control device 40, an input device 50 which is connected to the control device 40 and operates the control device 40, and a display device 60 which is also connected to the control device 40 and displays a video signal obtained in the control device 40.

The ultrasound endoscope 20 is inserted into a body cavity or the like, transmits ultrasound beam toward an observation target region, receives a reflected wave which is reflected by a boarder of an acoustic impedance of the observation target region, and obtains an echo signal. The control device 40 is connected to the ultrasound endoscope 20 with cables 21a and 21b via a connector 40a, controls transmission and reception of the ultrasound endoscope 20, and converts the obtained echo signal into a video signal which can be displayed.

The ultrasound endoscope 20 has an elongated probe which is inserted into a body cavity or the like, and a transducer group 30, that is, two transducer groups, a CLA (Curved Linear Array) scanning transducer group 31 and an ELR (Electrical Radial) scanning transducer group 32, which transmit and receive ultrasound beam.

The control device 40 includes a switching section 41 which selects either the transducer group 31 or 32 to switch to it, a transmission and reception processing section 42 which drives the selected transducer group 31 or 32 selected in the switching section 41, and performs wave detection of an echo signal and the like, an image generating section 44 which generates display image data from data obtained in the transmission and reception processing section 42, a switching section B46 which stores an image of either the transducer group 31 or 32, which is selected, in a CLA image memory 47 or an ELR image memory 48, which is the image memory of the transducer group 31 or 32, and a control section 45 which also controls the entire ultrasound diagnostic apparatus.

The display device 60 has two display areas. A CLA image stored in the CLA image memory 47 is displayed on a display area A60a, and an ELR image stored in the ELR image memory 48 is displayed on a display area B60b.

Further, the control device 40 includes a synthesis table 43. The transmission and reception section 42 gives a time difference to a drive signal to be transmitted to each transducer element (hereinafter, called a transducer) configuring the transducer group in accordance with the control data stored in the synthesis table 43. As a result, ultrasound having a phase difference corresponding to the time difference of the drive signal is transmitted from each of the transducers. By wavefront synthesis of the transmitted ultrasound having the phase difference, one ultrasound ray along a sound ray in a predetermined direction is synthesized. Each of the transducers which transmit ultrasound receives the echo signal that is the transmitted ultrasound which is reflected and returns, and the echo signals are synthesized to be a signal of one frame in the transmission and reception processing section 42 in accordance with the control data stored in the synthesis table 43 to be frame data for image generation.

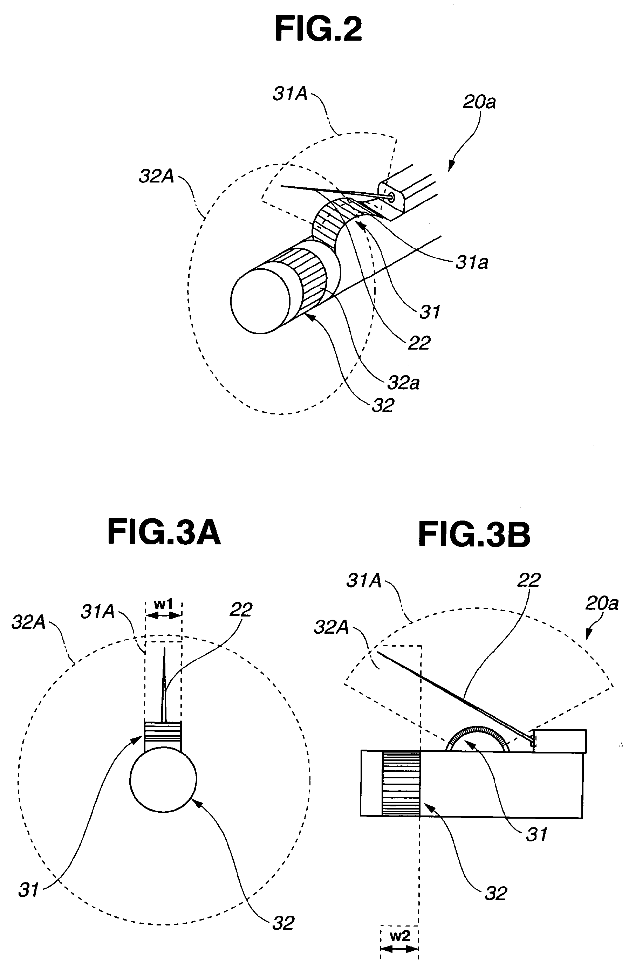

Fig. 2 is a perspective view of a distal end portion of a probe 20a of the ultrasound endoscope 20. The CLA scanning transducer group 31 scans within a surface parallel with a probe shaft in a sector form as shown by a scanning surface 31A. Specifically, respective transducers 31 a configuring the CLA scanning transducer group 31 are arranged in the form of a circular arc. Meanwhile, the ELR scanning transducer group 32 scans within a surface perpendicular to the probe shaft in a circular form as shown by a scanning surface 32A. Specifically, respective transducers 32a configuring the ELR scanning transducer group 32 are arranged in a circumferential form. Further, for example, a biopsy needle 22 for extracting a cell sample is caused to perform centesis along the CLA scanning surface 31A.

Fig. 3A is a front view of the distal end portion of the probe 20a of the present embodiment. Fig. 3B is a side view thereof. As shown in Figs. 3A and 3B, the CLA scanning surface 31A and the ELR scanning surface 32A are orthogonal to each other. It should be noted that the scanning surface 31 A and the scanning surface 32A have the depths corresponding to widths w1 and w2 of the respective transducers.

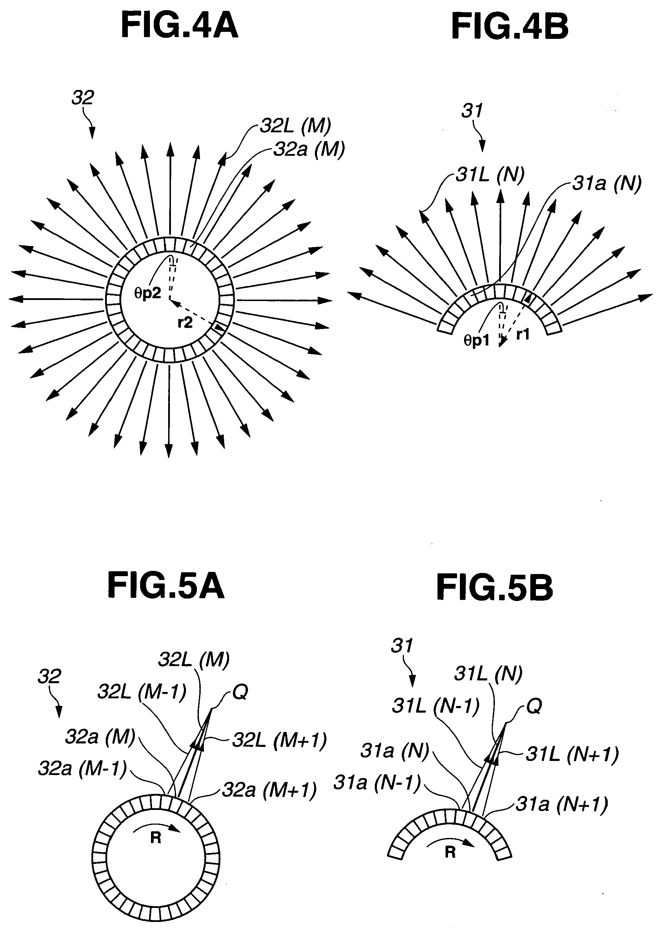

Fig. 4A shows a sectional view showing the sound rays of the ELR scanning transducer group 32 according to the present embodiment. Fig. 4B shows a sectional view showing the sound rays of the CLA scanning transducer group 31. In the ELR scanning transducer group 32, k_{ELR} of the transducers 32a are arranged in a circumferential form, the M^{th} transducer is set as 32a(M), and a sound ray which is transmitted with the transducer 32a(M) as a center is shown by 32L(M). Similarly, in the CLA scanning transducer group 31, k_{CLA} of the transducers 31a are arranged in an arc form, the N^{th} transducer is set as 31 a(N), and a sound ray which is transmitted with the transducer 31 a(N) as a center is shown by 31L(N).

In the present embodiment, a radius of curvature r1 of the CLA scanning transducer group 31 and a radius of curvature r2 of the ELR scanning transducer group 32 are the same.

Therefore, in the CLA scanning transducer group 31 and the ELR scanning transducer group 32, the relative transmission direction of the ultrasound of each of the transducers is the same. Specifically, the relationship of the ultrasound transmission direction of a certain transducer and the ultrasound transmission direction of a transducer adjacent to that transducer is constant.

Further, in the present embodiment, an arrangement pitch θp1 of each of the transducers 31a of the CLA scanning transducer group 31 and an arrangement pitch θp2 of each of the transducers 32a of the ELR scanning transducer group 32 are the same.

Fig. 5A is a sectional view showing sound ray synthesis of the ELR scanning transducer group 32 according to the present embodiment. Fig. 5B is a sectional view showing sound ray synthesis of the CLA scanning transducer group 31. Here, transmission and reception of ultrasound of the transducers is performed while moving in sequence in the directions of R shown in Figs. 5A and 5B.

Of a plurality of arranged transducers, high voltage pulses having adjusted delay amounts respectively are applied to some of a plurality of adjacent transducers, ultrasounds of burst waves having adjusted phases are transmitted from the plurality of transducers due to shift of timing of application of high voltage pulses, and by overlapping of the ultrasounds differing in phase, one sound ray is formed. Like this, for formation of a sound ray, a number of transducers are not used individually, but are jointly used in sequence.

For example, on an occasion of transmission of a J^{th} transducer, control of causing an adjacent (J-1)^{th} transducer to perform weak transmission little earlier than the J^{th} transducer, and causing a (J+1)^{th} transducer to perform weak transmission a little later than the J^{th} transducer is conducted. Data for the control is data for sound ray synthesis, and is stored in the sound ray synthesis table.

In Fig. 5A, a sound ray 32L(M-1) from a transducer 32a(M-1) and a sound ray 32L(M+1) from a transducer 32a(M+1) are synthesized with a sound ray from a transducer 32a(M), and one sound ray 32L(M) with a focus point of Q is formed. Similarly, in Fig. 5B, a sound ray 31L(M-1) from a transducer 31a(M-1), and a sound ray 31L(M+1) from a transducer 31a(M+1) are synthesized with a sound ray from a transducer 31 a(M), and one sound ray 31 L(M) with a focus point of Q is formed.

Then, the ultrasound transmitted into a subject (not illustrated) travels inside the examined boy while being reflected at each point inside the subject, and the ultrasound reflected at each point inside the subject is received by some of the plurality of adjacent transducers which are arranged and is converted into a reception signal. The reception signals are relatively delayed by the adjusted delay amounts and added to each other, whereby a frame signal expressing a reception sound ray extending inside the subject along the sound ray is formed. Specifically, in Figs. 5A and 5B, on the occasion of reception of reflected waves of ultrasounds, the reception signals received by three adjacent transducers are synthesized, and one frame signal is obtained.

Then, by scanning the transducers which perform transmission and reception in sequence while moving in the R direction, the ultrasound image along the scanning surface can be obtained.

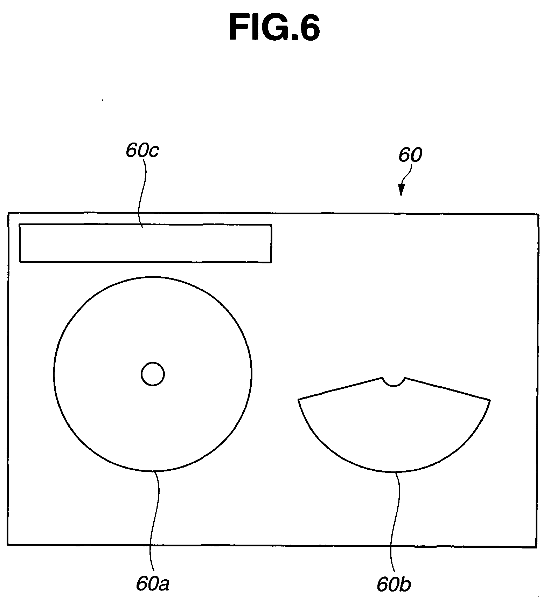

Fig. 6 shows a display example of the ultrasound image displayed on the display device 60 of the present embodiment. In Fig. 6, on the display screen 60, an ultrasound image 60a which the ELR scanning transducer group 32 scans, and an ultrasound image 60b which the CLA scanning transducer group 31 scans are simultaneously displayed. Therefore, an operator can perform diagnosis based on both the images. Further, the display screen 60 may have an area 60c on which the diagnosis conditions or the like of the diagnostic apparatus are displayed.

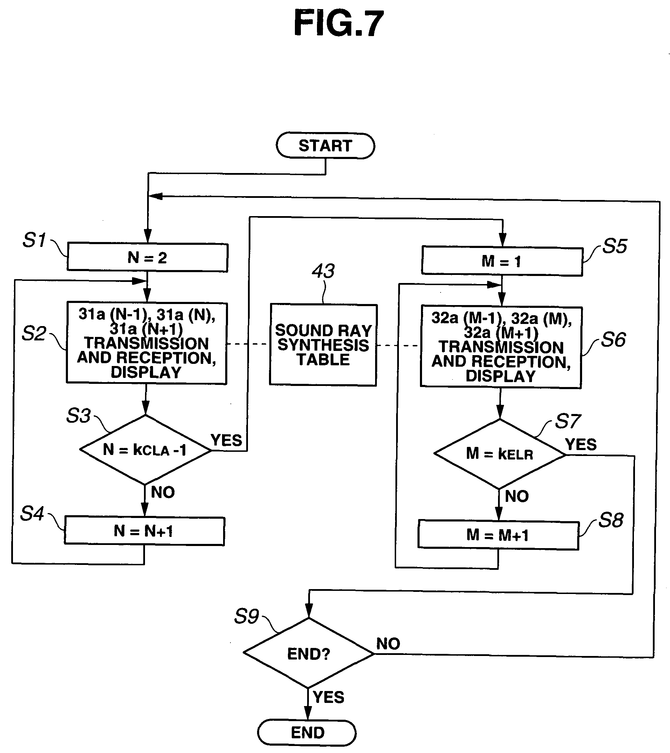

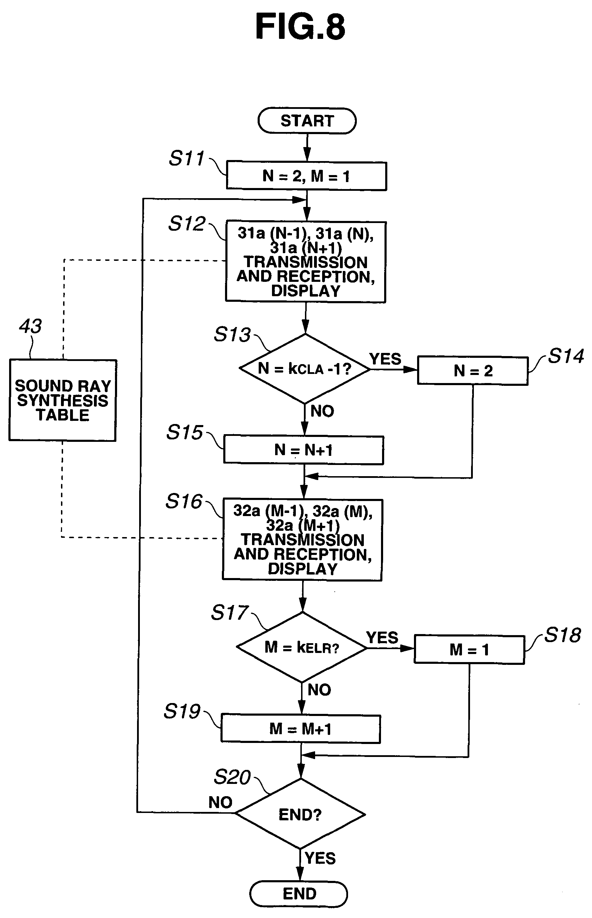

Figs. 7 and 8 are flowcharts showing a scanning procedure of each of the transducers configuring the transducer groups of the present embodiment. Figs. 7 and 8 show examples of the ultrasound diagnostic apparatus having the CLA scanning transducer group 31 configured by K_{CLA} of the transducers 31a, and the ELR scanning transducer group 32 configured by k_{ELR} of the transducers 32a.

In the scanning procedure shown in Fig. 7, after scanning and display of the entire CLA scanning surface by all k_{CLA} of the CLA scanning transducers 31a are completed, scanning and display of the entire ELR scanning surface by all K_{ELR} of the ELR scanning transducers 32a are performed. Then, scanning and display of the entire CLA scanning surface, and scanning and display of the entire ELR scanning surface are repeated.

Specifically, when scanning is started, a set value N for selecting the transducers to perform scanning first from the CLA surface scanning transducers 31a is initialized (S1). Then, the transmitting and receiving operation of the three adjacent transducers 31a(N-1), 31a(N) and 31a(N+1) is performed, and an image of one frame is displayed on the display device (S2). For the control on the occasion of the transmitting and receiving operation, the control data stored in the sound ray synthesis table 43 is used.

Next, in order to obtain the next frame image by using the same CLA scanning transducer group 31, one is added to the set value N (S4). Then, scanning by the next CLA surface scanning transducer 31 a is performed. At a point of time when the processing up to a transducer 31a (K_{CAL}-1) at an end of the transducer group 31 is completed (S3), specifically, at a point of time when scanning of the entire CLA scanning surface is completed, scanning of the ELR scanning surface is started.

In scanning of the ELR scanning surface, a set value M for selecting transducers to perform scanning first from the ELR surface transducers 32a is also initialized (S5). Then, the transmitting and receiving operation of the three adjacent transducers 32a(M-1), 32a(M) and 32a(M+1) is performed, and an image of one frame is displayed on the display device (S6). For control on the occasion of the transmitting and receiving operation, the control data stored in the same sound ray synthesis table 43 as at the time of scanning of the CLA scanning surface is also used.

Next, in order to obtain the next frame image by using the same ELR scanning transducer group 32, one is added to the set value M (S8). Then, scanning by the next ELR surface scanning transducer 32a is performed. If there is no instruction to end from an operator at a point of time when processing through an ELR surface scanning transducer 32a (k_{ELR}) at an end of the transducer group 32 is completed (S7), that is, at a point of time when scanning of the entire ELR scanning surface is completed (S9), scanning of the CLA scanning surface is started again. Then, the operation from S 1 is repeatedly performed.

On the other hand, in a scanning procedure of the transducer groups shown in Fig. 8, after scanning and display of a sound ray 31 L(N) by using the three transducers 31a(N-1), 31a(N) and 31a(N+1) out of K_{CLA} of the CLA scanning transducers 31 a, scanning and display of a sound ray 32L(M) are performed by using the three transducers 32a(M-1), 32a(M) and 32a(M+1) out of k_{ELR} of the ELR scanning transducers 32a. Then, scanning and display of a sound ray 31L(N+1) and scanning and display of a sound ray 32L(M+1) are further performed in sequence.

Specifically, when scanning is started, the set values N and M for selecting transducers to perform scanning first are initialized (S11). Then, transmitting and receiving operation of the three adjacent transducers 31a(N-1), 31a(N) and 31a(N+1) of the CLA scanning transducer group 31 is performed, and an image of one frame is displayed on the display device (S12). For control on the occasion of transmitting and receiving operation, the control data stored in the sound ray synthesis table 43 is used. If the scanned and displayed transducer 31 a is not the transducer 31a(k_{CAL}-1) at the end of the CLA transducer group 31 (S13), one is added to the set value N (S 15). If the scanned and displayed transducer 31 a is the transducer 31 a(k_{CAL}-1) (S13), N is initialized (S 14) in preparation for the next scanning and display.

Next, transmitting and receiving operation of the three adjacent transducers 32a(M-1), 32a(M) and 32a(M+1) of the ELR scanning transducer group 32 is performed, and an image of one frame is displayed on the display device (S16). For control on the occasion of the transmitting and receiving operation, the control data stored in the same sound ray synthesis table 43 as at the time of scanning of the CLA scanning surface is used. If the scanned and displayed transducer 32a is not the transducer 32a (k_{ELR}) at the end of the ELR transducer group 32 (S17), one is added to the set value M (S19). If the scanned and displayed transducer 32a is the transducer 32a(k_{ELR}) (S 17), M is initialized (S18) in preparation for the next scanning and display. Then, until the instruction to end is given from the operator (S20), the operation from S 12 is repeatedly performed.

In the scanning procedure shown in the flowchart of Fig. 8, new frame display is performed for the display device 60 for each one frame signal by the CLA scanning transducer group 31 or the ELR scanning transducer group 32. Specifically, the ultrasound diagnostic apparatus using the scanning procedure shown in the flowchart of Fig. 8 switches the transducer group to perform scanning each time one sound ray is scanned by scanning of a plurality of transducer groups. Therefore, as compared with the ultrasound diagnostic apparatus using the scanning procedure shown in Fig. 7, a time shift between the CLA scanning surface display 60b and the ELR scanning surface display 60a is eliminated, and an image which is more real-time can be displayed for the operator.

The transducer 31a(1) at the end of the CLA scanning transducer group 31 is only used for synthesis of the sound ray 31 L(2), and does not transmit a sound ray 31L(1). Similarly, the transducer 31a(k_{CLA}) at the other end of the CLA scanning transducer group 31 is only used for synthesis of the sound ray 31L(K_{CLA}-1), and does not transmit a sound ray 31L(k_{CLA}). On the other hand, a sound ray 32L(1) of the ELR scanning transducer group 32 is synthesized from three transducers that are transducers 32a(k_{ELR}), 32a(1) and 32a(2), and a sound ray 32L(k_{ELR}) is similarly synthesized from three transducers that are transducers 32a(k_{ELR}-1), 32a(k_{ELR}) and 32a(1).

Therefore, in the flowcharts of Figs. 7 and 8, the initial value of the set value N on the occasion of CLA scanning is two, and the transducer 31 which transmits the sound ray at the end of the CLA scanning transducer group 31 is the transducer 31a(k_{CAL}-1), whereas the initial value of the set value M on the occasion of ELR scanning is one, and the last transducer 32 of the ELR scanning transducer group 32 is the transducer 32a(k_{ELR}).

In order to obtain one sound ray and one frame data by cooperative operation of a plurality of transducers as described above, the control data such as drive control data for transmission and reception and parameters for processing the received data have to be obtained in advance and stored in the sound ray synthesis table. In order to share the sound ray synthesis table by a plurality of transducer groups, the sound ray directions of the respective transducers, that is, relative transmitting directions of ultrasounds need to correspond to each other between the respective transducer groups. In other words, the radiuses of curvature of the transducer groups which share the sound ray synthesis table have to be the same. If the transducer groups are not the transducer groups of such a special combination, the common sound ray synthesis table cannot be used, and common use by switching of the transducer groups by the switching section cannot be performed.

Further, the disposition spaces of the individual transducers of the transducer groups which share the table, that is, the pitch θp1 and the pitch θp2 preferably correspond to each other.

Further, in the above described embodiment, the example of synthesizing one sound ray from the three adjacent transducers is described, but one sound ray may be synthesized from the number of transducers exceeding three. Further, by conducting control using a pseudo sound ray, not only sound rays can be transmitted from the transducers at both ends of the CLA transducer group, but also the common sound ray synthesis table can be used even by the transducer groups differing in a pitch θp.

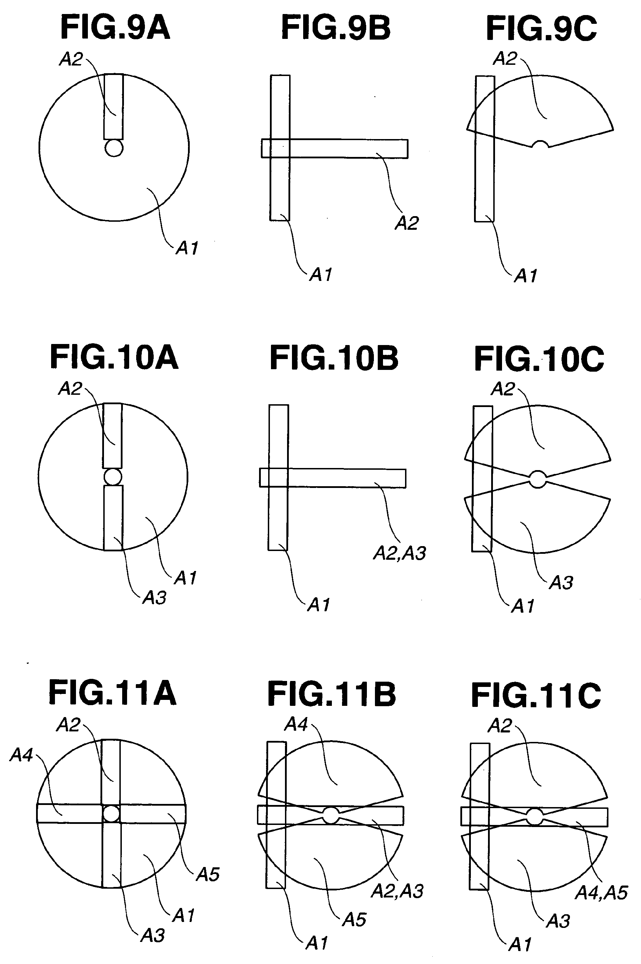

In the above described embodiment, the example in which one radial transducer group and one convex transducer group, that is, two transducer groups are used is described, but a plurality of transducer groups that are three or more can be used by being switched. Figs. 9A to 9C, 10A to 10C and 11A to 11C are views showing scanning surfaces in the ultrasound diagnostic apparatuses each having a plurality of transducer groups which scan within the surfaces orthogonal to each other. Figs. 9A, 10A and 11A are front views each observed in a direction perpendicular to a scope longitudinal direction. Figs. 9B, 10B and 11B are top views each observed from a top surface with respect to the scope longitudinal direction. Figs. 9C, 10C and 11C are side views each observed from a side surface with respect to the scope longitudinal direction.

Figs. 9A to 9C show an example of scanning two surfaces A1 and A2 by using one of each of the same radial transducer group and convex transducer group as the above described embodiment, that is, two transducer groups. On the other hand, Figs. 10A to 10C show an example of scanning three surfaces A1, A2 and A3 by using one radial transducer group and two convex transducer groups, that is, three transducer groups. Figs. 11A to 11C show an example of scanning five surfaces A1, A2, A3, A4 and A5 by using one radial transducer group and four convex transducer groups, that is, five transducer groups. Even in each of the embodiments in which the number of transducer groups is three or more, the basic configurations are the same as those of the embodiment in which the number of transducer groups is two.

In the ultrasound diagnostic apparatus using a plurality of electronic scanning type transducer groups, an ultrasound diagnostic apparatus in which by using the probe having the transducer group configured by a plurality of transducers arranged in a circumferential or arc form having a same radius of curvature, the sound ray synthesis table is shared, and one transmission and reception section and one image generating section and the like are usable by being switched is described.

However, by including a plurality of sets of transmission and reception sections, image generating sections and the like, the ultrasound diagnostic apparatus according to the embodiment of the present invention has the advantage of being continuously usable by switching a set of transmission and reception section, image generating section and the like to the other set of the transmission and reception section, image generating section and the like which are not out of order when the one set of the transmission and reception section, image generating section and the like is out of order. Especially when three or more transducer groups are used, the processing speed may become low when one set of a transmission and reception section, an image generating section and the like is shared, and a plurality of sets of transmission and reception sections, image generating sections and the like are preferably included.

Next, an ultrasound diagnostic apparatus according to another embodiment of the present invention will be described. In the ultrasound diagnostic apparatus according to the present embodiment, scanning of a plurality of transducer groups is in accordance with a sequence set in advance, and the transducer group to perform scanning is switched. Specifically, in the ultrasound diagnostic apparatus already described, for example, in the scanning procedure shown in the flowchart of scanning of the respective transducers configuring the transducer groups in Fig. 7, at the point of time when scanning of the entire CLA scanning surface is completed, scanning of the ELR scanning surface is started, and at the point of time when the scanning of the entire ELR scanning surface is completed, scanning of the CLA scanning surface is started. Specifically, at the point of time when scanning of the entire scanning surface of each of the transducer groups is completed, the transducer group to perform scanning is switched. Further, in the ultrasound diagnostic apparatus already described, in the scanning procedure shown in the flowchart of scanning of the respective transducers configuring the transducer groups of Fig. 8, for example, the transducer group to perform scanning is switched at every one frame signal by the CLA scanning transducer group 31 or the ELR scanning transducer group 32.

On the other hand, in the ultrasound diagnostic apparatus according to the different embodiment of the present invention, the transducer group to perform scanning is switched in accordance with the sequence which is set in advance. Further, the scanning image displayed on the display device 60 may be only the image of the transducer group which performs scanning.

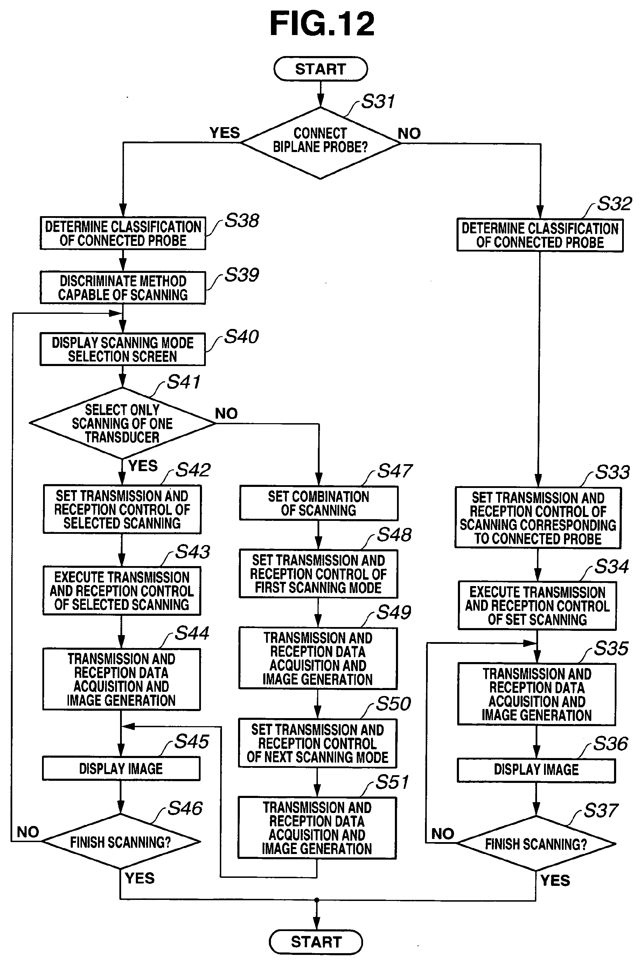

Hereinafter, the flow of the process of the ultrasound diagnostic apparatus according to the present embodiment will be described by using Fig. 12. Fig. 12 is a flowchart for explaining the flow of the process of the ultrasound diagnostic apparatus according to the present embodiment.

In the ultrasound diagnostic apparatus of the present embodiment, when the ultrasound endoscope 20 is connected to the control device 40, the control device 40 determines whether the connected ultrasound endoscope 20 is an endoscope probe having a plurality of transducer groups, for example, a biplane probe (step S31). When the connected probe is an ordinary probe, that is, a probe having only one transducer group, it is determined as No in step S31, and the process from step 32 is performed in sequence.

On the other hand, when it is determined as Yes in step S31, detailed classification of the connected probe is further determined (step S38). Then, a mode capable of scanning with the connected probe, for example, a radial mode, a convex mode, a linear mode or the like is determined (step S39).

Subsequently, in step S40, a selection screen for setting a scanning mode, that is, setting the transducer group to perform scanning in advance from a plurality of transducer groups is displayed on a display device 70 or the like. In the selection screen, the transducer group to perform scanning, the scanning sequence, the scanning switching timing (time) and the like can be set. By a scanning mode setting instruction of the operator from the selection screen, the control circuit 40 switches the transducer group for scanning in accordance with the sequence or the like set in advance.

In step S40, when only one transducer group is selected from a plurality of transducer groups, it is determined as Yes in step S41, and the process from step 42 is performed in sequence.

On the other hand, in step S41, when it is determined as No, combination for switching the transducer group to perform scanning is set in accordance with the sequence or the like set in advance in step S40 (step S47), and transmission and reception control of one transducer group to perform scanning first is set by the control device 40 (step S48). Subsequently, transmission and reception data acquisition and image generation of the one transducer group which performs scanning first are performed (step S49). Next, the control device 40 performs transmission and reception control setting of one transducer group which is set to perform scanning next (step S50) and performs transmission and reception data acquisition and image generation (step S51), and displays the image (step S45).

In each scanning, scanning is continuously performed until the instruction to stop scanning or the like is given from the operator.

In the ultrasound diagnostic apparatus of the present embodiment, scanning switching of a plurality of transducer groups can be set in advance as the operator desires. Therefore, the ultrasound diagnostic apparatus of the present embodiment is further excellent in operability in addition to the effect of the aforementioned ultrasound diagnostic apparatus according to the present invention has.

Having described the preferred embodiments of the invention referring to the accompanying drawings, it should be understood that the present invention is not limited to those precise embodiments and various changes and modifications thereof could be made by one skilled in the art without departing from the spirit or scope of the invention as defined in the appended claims.

## Claims

1. An ultrasound diagnostic apparatus, comprising:
an elongated probe;
a radial scanning transducer group (32) provided at the distal end portion of the probe (20a) and configured by a plurality of radial scanning transducers (32a) arranged in a circumferential form and which perform radial scanning in a surface perpendicular to a probe shaft of the probe (20a) in a circular form; and
a convex scanning transducer group (31) provided at the distal end portion of the probe (20a) and configured by a plurality of convex scanning transducers (31a) which perform scanning in a sector form within a surface parallel to the probe shaft and are arranged in an arc r form; **characterized in that** the convex scanning transducer group (31) has the pitch and the same radius of curvature as the radial scanning transducer group (32); and
**in that** the ultrasound diagnostic apparatus comprises a common sound ray synthesis table storing control data of the radial scanning transducer group (32) and the convex scanning transducer group (31).

2. The ultrasound diagnostic apparatus according to claim 1,
further comprising a display section (60) having a display screen capable of simultaneously displaying two ultrasound images,
wherein two ultrasound images scanned by the radial scanning transducer group (32) and the convex scanning transducer group (31) are simultaneously displayed on the display section (60).

3. The ultrasound diagnostic apparatus according to claim 1,
further adapted to perform scanning of the radial scanning transducer group (32) and scanning of the convex scanning transducer group (31) in accordance with a sequence set in advance, and to switch the transducer group to perform scanning.

4. The ultrasound diagnostic apparatus according to claim 1,
further adapted to switch the transducer group to perform scanning each time the radial scanning transducer group (32) and the convex scanning transducer group (31) scan one sound ray.

## Patentansprüche

1. Ultraschall-Diagnosegerät, das umfasst:
eine langgestreckte Sonde;
eine radiale Abtastwandlergruppe (32), die an dem distalen Endabschnitt der Sonde (20a) vorgesehen und durch eine Mehrzahl von radialen Abtastwandlern (32a) gebildet wird, die in einer Umfangsform angeordnet sind und die in einer zu einem Sondenschaft der Sonde (20a) senkrechten Oberfläche eine radiale Abtastung in einer Kreisform durchführen; und
eine konvexe Abtastwandlergruppe (31), die an dem distalen Endabschnitt der Sonde (20a) vorgesehen und durch eine Mehrzahl von konvexen Abtastwandlern (31a) gebildet wird, die in einer zu dem Sondenschaft parallelen Oberfläche eine Abtastung in einer Sektorform durchführen und in einer Kreisbogenform angeordnet sind;
**dadurch gekennzeichnet, dass** die konvexe Abtastwandlergruppe (31) das gleiche Abstandsmaß und den gleichen Krümmungsradius hat wie die radiale Abtastwandlergruppe (32); und
dadurch, dass das Ultraschall-Diagnosegerät eine gemeinsame Schallstrahl-Synthesetabelle umfasst, die Steuerdaten der radialen Abtastwandlergruppe (32) und der konvexen Abtastwandlergruppe (31) speichert.

2. Ultraschall-Diagnosegerät nach Anspruch 1,
das ferner einen Anzeigeabschnitt (60) umfasst, der einen Anzeigebildschirm hat, der dazu in der Lage ist, gleichzeitig zwei Ultraschallbilder anzuzeigen,
wobei zwei Ultraschallbilder, die durch die radiale Abtastwandlergruppe (32) und die konvexe Abtastwandlergruppe (31) abgetastet werden, gleichzeitig auf dem Anzeigeabschnitt (60) angezeigt werden.

3. Ultraschall-Diagnosegerät nach Anspruch 1,
das ferner dazu eingerichtet ist, eine Abtastung der radialen Abtastwandlergruppe (32) und eine Abtastung der konvexen Abtastwandlergruppe (31) gemäß einer vorab festgelegten Sequenz durchzuführen und die zur Durchführung einer Abtastung Wandlergruppe umzuschalten.

4. Ultraschall-Diagnosegerät nach Anspruch 1,
das ferner dazu eingerichtet ist, die Wandlergruppe zur Durchführung einer Abtastung jedes Mal umzuschalten, wenn die radiale Abtastwandlergruppe (32) und die konvexe Abtastwandlergruppe (31) einen Schallstrahl abtasten.

## Revendications

1. Appareil de diagnostic à ultrasons, comprenant :
une sonde allongée ;
un groupe de transducteurs de numérisation radiale (32) prévu sur la partie d'extrémité distale de la sonde (20a) et configuré par une pluralité de transducteurs de numérisation radiale (32a) agencés en forme circonférentielle et qui effectuent une numérisation radiale dans une surface perpendiculaire à une tige de sonde de la sonde (20a) dans une forme circulaire ; et
un groupe de transducteurs de numérisation convexe (31) prévu sur la partie d'extrémité distale de la sonde (20a) et configuré par une pluralité de transducteurs de numérisation convexe (31a) qui effectuent une numérisation dans une forme sectorielle à l'intérieur d'une surface parallèle à la tige de sonde et sont agencés en une forme arquée ;
**caractérisé en ce que** le groupe de transducteurs de numérisation convexe (31) présente le même pas et le même rayon de courbure que le groupe de transducteurs de numérisation radiale (32) ; et
**en ce que** l'appareil de diagnostic à ultrasons comprend une table de synthèse de rayon sonore commune conservant des données de commande du groupe de transducteurs de numérisation radiale (32) et du groupe de transducteurs de numérisation convexe (31).

2. Appareil de diagnostic à ultrasons selon la revendication 1,
comprenant en outre une section d'affichage (60) présentant un écran d'affichage en mesure d'afficher simultanément deux images d'ultrasons,
dans lequel deux images d'ultrasons numérisées par le groupe de transducteurs de numérisation radiale (32) et le groupe de transducteurs de numérisation convexe (31) sont affichées simultanément sur la section d'affichage (60).

3. Appareil de diagnostic à ultrasons selon la revendication 1,
adapté en outre pour effectuer une numérisation du groupe de transducteurs de numérisation radiale (32) et une numérisation du groupe de transducteurs de numérisation convexe (31) en fonction d'un jeu de séquences à l'avance et pour basculer le groupe de transducteurs pour qu'il effectue la numérisation.

4. Appareil de diagnostic à ultrasons selon la revendication 1,
adapté en outre pour basculer le groupe de transducteurs pour qu'il effectue la numérisation à chaque fois que le groupe de transducteurs de numérisation radiale (32) et le groupe de transducteurs de numérisation convexe (31) numérisent un rayon sonore.
